(19) **Europäisches Patentamt / European Patent Office / Office européen des brevets**

(11) **EP 4 523 674 B1**

(12) **EUROPEAN PATENT SPECIFICATION**

(45) Date of publication and mention of the grant of the patent:
**15.10.2025 Bulletin 2025/42**

(21) Application number: **23731726.8**

(22) Date of filing: **12.05.2023**

(51) International Patent Classification (IPC):
*A61K 8/31* (2006.01)  *A61K 8/34* (2006.01)
*A61K 8/36* (2006.01)  *A61K 8/44* (2006.01)
*A61K 8/49* (2006.01)  *A61Q 17/04* (2006.01)
*A61K 8/19* (2006.01)

(52) Cooperative Patent Classification (CPC):
**A61K 8/498; A61K 8/19; A61K 8/31; A61K 8/347; A61K 8/361; A61K 8/44; A61K 8/494; A61Q 17/04;** A61K 2800/58

(86) International application number:
**PCT/ES2023/070306**

(87) International publication number:
**WO 2023/218115 (16.11.2023 Gazette 2023/46)**

(54) **COMPOSITION FOR PROMOTING PHOTOPROTECTION AND CUTANEOUS REGENERATION THROUGH SEQUENTIAL PHOTONIC TRANSFER (SPT)**

ZUSAMMENSETZUNG ZUR FÖRDERUNG DES LICHTSCHUTZES UND DER HAUTREGENERATION DURCH SEQUENTIELLEN PHOTONISCHEN TRANSFER (SPT)

COMPOSITION DESTINÉE À FAVORISER LA PHOTOPROTECTION ET LA RÉGÉNÉRATION CUTANÉE PAR TRANSFERT PHOTONIQUE SÉQUENTIEL (TFS)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.05.2022 ES 202230422**

(43) Date of publication of application:
**19.03.2025 Bulletin 2025/12**

(73) Proprietor: **ESPADA REGALADO, Jesús 28034 Madrid (ES)**

(72) Inventors:
• **CALVO-SÁNCHEZ, María Inmaculada 28034 Madrid (ES)**
• **FERNÁNDEZ-MARTOS, Sandra 28034 Madrid (ES)**
• **ESCOBAR, Marta 28034 Madrid (ES)**
• **PINACHO-PÉREZ, Aida 28034 Madrid (ES)**
• **ESPADA REGALADO, Jesús 28034 Madrid (ES)**

(74) Representative: **TRBL Intellectual Property Plaza de Castilla 3, 7°A 28046 Madrid (ES)**

(56) References cited:
WO-A1-2008/106983    WO-A1-2019/186544
WO-A1-2020/249747    WO-A1-2021/207182
WO-A2-03/039452    WO-A2-2009/095568

• DATABASE GNPD [online] MINTEL; 16 December 2020 (2020-12-16), ANONYMOUS: "Facial Oil", XP093081594, retrieved from https://www.gnpd.com/sinatra/recordpage/8318243/ Database accession no. 8318243
• DATABASE GNPD [online] MINTEL; 15 July 2021 (2021-07-15), ANONYMOUS: "Hemp Body Oil", XP093081697, retrieved from https://www.gnpd.com/sinatra/recordpage/8862519/ Database accession no. 8862519
• DATABASE GNPD [online] MINTEL; 24 June 2021 (2021-06-24), ANONYMOUS: "Mauvaise Herbe Indica Oil", XP093081712, retrieved from https://www.gnpd.com/sinatra/recordpage/8736877/ Database accession no. 8736877

**Description**

**FIELD OF THE INVENTION**

The present invention falls within the field of biotechnology and relates to compositions that combine organic compounds capable of absorbing ultraviolet (UV) sunlight and converting it into red light, thereby promoting broad-spectrum photoprotection against solar radiation and cutaneous regeneration. The photoprotective effect is due to the compounds in these compositions that can absorb UV light, while the regenerative effect is attributed to the final emission of red light from the combination of compounds, which, in turn, transiently activates the production of non-lethal levels of reactive oxygen species (ROS) in the tissue, sufficient to stimulate the homeostasis and regeneration programs within it.

**BACKGROUND**

The proper care and maintenance of the skin in response to a multitude of pathological conditions showing a significant impact on the population (melanoma and non-melanoma skin cancer, bacterial and viral infections, aggressions by physical/chemical agents, chronic ulcers in elderly and/or diabetic individuals, long-lasting surgical wounds, psoriasis, atopic dermatitis, and many others) represent a fundamental challenge for the healthcare system with enormous social and economic repercussions, especially concerning the elderly population. Skin-related conditions involving adjacent organs such as hair follicles or sweat glands, as well as the gradual tissue degradation associated with aging or the continuous exposure to environmental pollutants, while not always posing a direct threat to the patient's health, also have a significant social impact and an increasingly growing market share. Noteworthy, the continuous exposure of the skin to solar radiation, resulting in a cumulative tissue damage, may result in severe pathologies such as melanoma skin cancer. In this context, and due to its enormous social impact, the skincare market in general, both in clinical and cosmetic aspects, is a sector showing sustained growth worldwide, with an expected exponential increase in the coming years. Within the biotechnological facet of this market, the highest growth projections are seen in cosmetics and regenerative medicine with dermocosmetic and clinical/ therapeutic applications. In this field, many of the therapies/ technologies/ products currently available on the market for treating a significant group of skin lesions and conditions, both clinically and cosmetically, have significant deficiencies and functional limitations that critically affect their actual efficacy, thus defining new and specific niches for R&D.

At the clinical level:

- Healing of long-lasting cutaneous lesions. The economic cost of chronic ulcers in elderly or diabetic individuals, long-lasting surgical or domestic wounds, amounts to billions of euros, constituting approximately 8-10% of the total healthcare expenditure, a constantly increasing figure due to population aging. There is no effective curative treatment for this type of lesion, with hospitalization time (50%) and nursing care expenses (30-35%) being the major economic burdens for the system.

At the cosmetic level:

- High-efficiency and eco-sustainable sun protection and anti-aging cutaneous regeneration based on natural compounds. One of the current priority trends in the sector is environmental respect and the use of natural/organic compounds. Most current sunscreens contain physical filters (e.g., titanium dioxide) or chemical filters (e.g., oxybenzone, avobenzone, octisalate, octocrylene, homosalate, octinoxate), synthetic compounds that can affect both the patient and the environment. On the other hand, the objective of regenerating and anti-aging compositions is the simultaneous protection and repair of the skin, as well as increasing its firmness and smoothness. The compositions of this kind available in the market are mostly simple moisturizing creams containing various types of additional molecules, typically synthetic, such as retinoids, coenzyme Q10, antioxidants, growth factors, collagens, alpha and beta hydroxy acids, or peptides like acetyl hexapeptide-8 (Argireline™) or matrikines (Matrixyl®). In most cases, the effect of these creams on skin repair and regeneration is limited primarily due to the low penetrability of the theoretical active ingredients through the epidermal barrier, or they penetrate but are rapidly degraded, or they penetrate but are modified, thus losing effectiveness.

In these two niches, there is clearly an opportunity for innovation and development of competitive products that address essential needs for a wide sector of the population. In both cases, the underlying biological processes mainly involve the homeostatic maintenance and regeneration of the skin, which depend mainly on the regulated activity of tissue stem cells. Therefore, one of the most promising lines of development at the therapeutic or cosmetic level in dermatology is the development of new technologies that allow for effective functional regulation of skin stem cells.

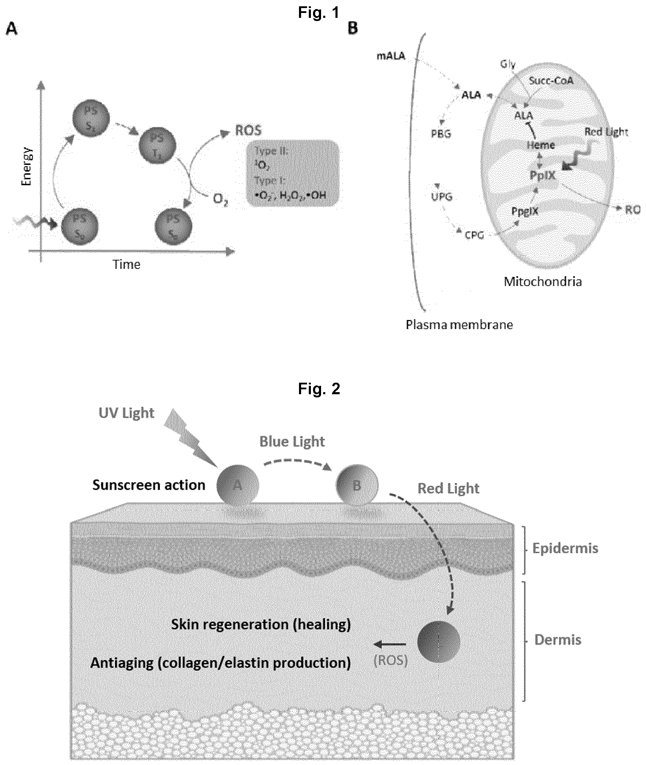

On the other hand, Reactive Oxygen Species (ROS) are highly reactive and unstable molecular intermediates that result from the reduction of molecular oxygen ($O_2$) to form water ($H_2O$) during aerobic metabolism in mammals. In all biological systems, the production of ROS exhibits a hormetic effect, meaning that their accumulation above a critical threshold is toxic, while at concentrations below this threshold, they can function as signaling molecules. Thus, the excessive and recurrent accumulation of these compounds in cells and tissues induces oxidative stress, which is causally involved in the development of multiple diseases, including cancer, and the aging process. However, in recent years, it has been demonstrated that under normal physiological conditions, low levels of ROS can indeed play essential roles as second messengers, being involved in the regulation of physiological processes such as cell proliferation, differentiation, and survival. In this context, it has been shown in recent years that ROS act as second messengers that regulate molecular mechanisms in the homeostasis and regeneration processes of mammalian skin. Thus, the controlled activation of the production of non-lethal levels of ROS in the tissue promotes the activation of proliferation and differentiation programs of resident stem cell niches, stimulating physiological processes such as burn and wound healing or hair growth. Such a controlled activation of ROS in the tissue may be carried out through a photodynamic process using a photosensitive or photosensitizer compound and light of an appropriate wavelength to produce ROS from molecular oxygen (Figure 1A). Specifically, this photodynamic process aims to stimulate the activation of a controlled production of ROS by the endogenous photosensitizer Protoporphyrin IX (PpIX), present in all eukaryotic cells. This photodynamic process requires the incorporation of PpIX precursors, such as the natural amino acid 5-aminolevulinic acid (ALA) or its methylated precursor, methyl-aminolevulinate (m-ALA), into the biological substrate in order to increase the basal cellular levels of PpIX and the subsequent irradiation with red light that specifically photosensitizes PpIX to produce ROS (Figure 1B). The combination of an appropriate concentration of precursors and light dosage allows for the controlled activation of cutaneous homeostasis and regeneration mechanisms in in vivo tissues.

Photodynamic processes have been described in the art.

For example, WO 2009/095568 A2 discloses a photodynamic skin rejuvenation therapy which makes use of a composition comprising a combination of chlorophyll A/B or chlorophylline and optionally aminolevulinic acid.

WO 2008/106983 A1 discloses a photodynamic treatment of skin using a composition comprising 5-aminolevulinic acid.

A critical limitation of this photodynamic process for implementing processes/methods or developing products to promote cutaneous homeostasis and regeneration at a cosmetic or pharmacological level is the need for an autonomous source of red light applied to the tissue to intracellularly photo-stimulate ROS production based on PpIX since the red-light radiation from the sun is not powerful enough to activate the process.

## DESCRIPTION OF THE INVENTION

The present invention is based on a process of sequential photonic transfer (SPT) between organic compounds, whereby a first organic compound absorbs photons of a specific wavelength and subsequently emits photons of a different wavelength, typically longer than the initial photon, which can then be absorbed by an adjacent second organic compound to emit even longer-wavelength photons. The chemical structure of each compound determines the corresponding ranges of photon absorption and emission. Depending on their chemical characteristics, a specific combination of organic compounds can sequentially couple the absorption and emission of photons of different wavelengths. In the present invention, the SPT process allows for the absorption of incident ultraviolet light from solar radiation, specifically UVB/UVA (between 280 and 400 nm), by a first organic compound, and the subsequent emission by the first organic compound of blue/green light photons (450-570 nm), these blue/green light photons being further absorbed to convert into red light photons (between 620 and 780 nm). This conversion mechanism from ultraviolet light photons to red light photons prevents excessive incidence of UVB/UVA rays on the skin, thus achieving a photoprotective effect.

Furthermore, the present invention contemplates the coupling of an SPT mechanism, where ultraviolet light photons (UVB/UVA) are initially absorbed, and red light photons are finally emitted with the transient activation of endogenous ROS production in biological systems, particularly mammalian cells and tissues, through the intracellular photo-stimulation of a photosensitive/photosensitizer compound by red light generated in the SPT process (Figure 2). In the context of the present invention, a "photosensitive/photosensitizer compound" refers to a compound capable of producing ROS in the presence of oxygen when irradiated with light of an appropriate wavelength, which can be administered externally (exogenous photosensitizer) or produced by the organism itself from a precursor (endogenous photosensitizer). An appropriate photosensitive/photosensitizer compound for use in the invention is Protoporphyrin IX (PpIX) or its precursors. This second mechanism of sequential photonic transfer in the skin allows for the regulation and stimulation of physiological functions related to cutaneous homeostasis and regeneration. Thus, a second mechanism enables sequential photonic transfer in the skin, utilizing UVB/UVA light photons from solar radiation (the most energetic ones that reach the Earth's surface) to produce a high number of red light photons on the epidermis's surface, reaching sufficient irradiance to reach cells in the inner layers of the skin and photo-stimulate PpIX, thereby activating the production of non-lethal ROS signals in the tissue.

Thus, in a first aspect, the invention relates to a composition capable of sequential photonic transfer (SPT), comprising at least one organic compound capable of absorbing UVB/A light (280-400 nm) and emitting blue/green light (450-570 nm), at least one organic compound capable of absorbing blue/green light (450-570 nm) and emitting red light (620-780 nm), and at least one photosensitive compound or a precursor thereof capable of absorbing red light and stimulating the production of ROS.

According to the present invention, the at least one organic compound capable of absorbing UVB/A light (280-400 nm) and emitting blue/green light (450-570 nm) is selected from the group consisting of polyphenols, flavonoids, cannabinoids, and combinations thereof. Flavonoids are polyphenolic compounds that have a general chemical structure with a 15-carbon skeleton containing ketones, where the general structure consists of two phenyl rings and a heterocyclic ring that includes an embedded oxygen. This chemical structure defines the spectral absorption and emission characteristics of these compounds. According to IUPAC nomenclature, flavonoids can be classified into bioflavonoids, isoflavonoids, and neoflavonoids. In the context of the invention, bioflavonoids (also known simply as flavonoids, including flavones, flavonols, flavonones, and anthocyanidins) are derivatives of a 2-phenylchromen-4-one (2-phenyl-1,4-benzopyrone) structure. In embodiments of the invention, bioflavonoids are selected from the group consisting of luteolin, apigenin, tangeritin, quercetin, morin, kaempferol, myricetin, fosetin, galangin, isorhamnetin, pachypodol, rhamnazin, pironoflavonols, furanoflavonols, hesperetin, naringenin, eriodictyol, homoeriodictyol, taxifolin, anthocyanidin, cyanidin, delphinidin, malvidin, pelargonidin, peonidin, petunidin, flavan-3-ol, flavan-4-ol, flavan-3,4-diol, proanthocyanidins. In the context of the invention, isoflavonoids are derivatives of the 3-phenylchromen-4-one (3-phenyl-1,4-benzopyrone) structure. In embodiments of the invention, isoflavonoids are selected from the group consisting of genistein, daidzein, glycitein, equol, lonchocarpin, laxiflorin, phytoestrogens. In the context of the invention, neoflavonoids are derivatives of the 4-phenylcoumarin (4-phenyl-1,2-benzopyrone) structure. In embodiments of the invention, neoflavonoids are selected from the group consisting of neoflavones (calophyllolide), neoflavanes (dalbergichromene), coutareagenin, dalbergin, nivetin. In the context of the present invention, cannabinoids, also called phytocannabinoids, are a mixed group of polyketide compounds, derived from prenylated malonyl-CoA and hexanoyl-CoA units prenylated with geranyl phosphate, with a main characteristic multiring terpenophenolic C21 skeleton. This general chemical structure delimits the spectral characteristics of absorption and emission of these compounds. The nomenclature applies to parental cannabinoids, cannabinoid derivatives, and transformation products. Phytocannabinoids can be classified into 11 subclasses of cannabinoids: cannabichromene (CBC), cannabidiol (CBD), canna-bielsoin (CBE), cannabigerol (CBG), cannabicyclol (CBL), cannabinol (CBN), cannabinodiol (CBND), cannabitriol (CBT), (-)-$\Delta$8-trans-tetrahydrocannabinol ($\Delta$8-THC), (-)-$\Delta$9-trans-tetrahydrocannabinol ($\Delta$9-THC), and mixed-type cannabinoids. In particular embodiments of the invention, the at least one organic compound capable of absorbing UVB/A light (280-400 nm) and emitting blue/green light (450-570 nm) is selected from the group consisting of morin, quercetin, cannabidiol, cannabinol, cannabichromene, and combinations thereof.

On the other hand, according to the present invention, the at least one organic compound capable of absorbing blue/green light (450-570 nm) and emitting red light (620-780 nm) is selected from the group consisting of carotenoids, xanthenes, chlorophylls, oleic acids, and combinations thereof. In the context of the present invention, carotenoids are tetraterpenoids that have a general chemical structure of a polyene hydrocarbon chain consisting of 9-11 bonds that may terminate in derivatives of the benzene ring. This general chemical structure delimits the spectral characteristics of absorption and emission of these compounds. The length of the polyene tail determines the wavelength of the absorbed and emitted light. In the context of the invention, carotenoids can be selected from carotenes and xanthophylls. Carotenes are non-oxygenated carotenoids that consist of a polyunsaturated hydrocarbon skeleton with 40 carbon atoms, a variable number of hydrogen atoms, and no other elements, such as alpha-carotene, beta-carotene, gamma-carotene, delta-carotene, lycopene. Xanthophylls are carotenoids that contain oxygen, such as lutein, zeaxanthin, neoxanthin, violaxanthin, flavoxanthin, alpha and beta-cryptoxanthin. In the context of the present invention, xanthenes are heterocyclic compounds derived from the xanthene backbone and consist of a xanthyl or di-benzo-g-pyrane core as the chromophore core with an amino or hydroxyl meta to the oxygen. This chromophoric structure delimits the spectral characteristics of absorption and emission of these compounds. Xanthenes can be selected from fluoresceins, eosins, and rhodamines. In the context of the present invention, chlorophylls consist of a chlorin ring, with its four nitrogen atoms surrounding a central magnesium atom, which can have different side chains and a hydrocarbon tail formed by a phytol ester. In chlorophylls c1 and c2, the chlorin ring is replaced by a porphyrin ring. The chloro group further delimits the spectral characteristics of absorption and emission of these compounds. In particular embodiments of the invention, chlorophylls can be selected from chlorophylls A, B, C1, C2, D, and F. In the context of the present invention, oleic acid is a fatty acid classified as an omega-9 monounsaturated fatty acid, abbreviated with a lipid number of 18:1 cis-9, and a major product of $\Delta$9 desaturase. More particularly, in embodiments of the invention, the at least one organic compound capable of absorbing blue/green light (450-570 nm) and emitting red light (620-780 nm) is selected from the group consisting of chlorophyll A, chlorophyll B, chlorophyll C1, chlorophyll C2, chlorophyll D, chlorophyll F, and combinations thereof.

In a particular embodiment of the invention, the composition comprises at least one organic compound capable of absorbing UVB/A light (280-400 nm) and emitting blue/green light (450-570 nm), and at least one organic compound capable of absorbing blue/green light (450-570 nm) and emitting red light (620-780 nm) that are chosen from the following combinations: chlorophyll A and morin; or chlorophyll A and quercetin; or chlorophyll A and cannabidiol; or chlorophyll A and cannabichromene; or chlorophyll A, morin, and cannabidiol; or chlorophyll A, morin, and cannabichromene; or chlorophyll A, morin, cannabidiol, and cannabichromene; or chlorophyll A, quercetin, and cannabidiol; or chlorophyll A, quercetin, and cannabichromene; or chlorophyll A, quercetin, cannabidiol, and cannabichromene.

According to the invention, the composition also comprises at least one photosensitive compound or a precursor thereof capable of absorbing red light and stimulating the production of ROS. In particular embodiments of the invention, the at least one photosensitive compound or precursor thereof capable of absorbing red light and stimulating the production of ROS is Protoporphyrin IX (PpIX), 5-aminolevulinic acid (ALA), and/or methyl-aminolevulinate (m-ALA). Thus, in a particular embodiment of the invention, the composition comprising at least one organic compound capable of absorbing UVB/A light (280-400 nm) and emitting blue/green light (450-570 nm), at least one organic compound capable of absorbing blue/green light (450-570 nm) and emitting red light (620-780 nm), and at least one photosensitive compound or precursor thereof capable of absorbing red light and stimulating the production of ROS is a composition comprising: morin and/or cannabidiol; chlorophyll A; and methyl-aminolevulinate (m-ALA).

The composition of the invention comprises, in additional embodiments, at least one additional sunscreen agent, of physical/mineral and/or chemical type, where the physical/mineral sunscreen agent can be, non-limitingly, zinc oxide or titanium dioxide, and where the chemical sunscreen agent can be, non-limitingly, oxybenzone, avobenzone, octisalate, octocrylene, homosalate, and/or octinoxate. Furthermore, in particular embodiments of the invention, the composition does not comprise lawsone.

In particular embodiments of the present invention, the composition is incorporated into a vehicle system or a cosmetically or pharmaceutically acceptable sustained release system selected from the group consisting of liposomes, mixed liposomes, oleosomes, niosomes, ethosomes, milicapsules, microcapsules, nanocapsules, sponges, cyclodextrins, vesicles, micelles, mixed micelles of surfactants, phospholipid-surfactant mixed micelles, millispheres, microspheres, nanospheres, lipospheres, microemulsions, nanoemulsions, miniparticles, miliparticles, microparticles, nanoparticles, solid lipid nanoparticles, and nanostructured lipid carriers. In other particular embodiments of the present invention, the composition is presented in a formulation selected from the group consisting of creams, multiple emulsions, anhydrous compositions, aqueous dispersions, oils, milks, balms, foams, lotions, gels, cream gels, hydroalcoholic solutions, hydroglycolic solutions, hydrogels, liniments, serums, soaps, shampoos, conditioners, serums, ointments, mousses, pomades, powders, bars, pencils, sprays, and aerosols. In yet other particular embodiments of the present invention, the composition is incorporated into a product selected from the group consisting of sunscreen, under-eye correctors, foundations, makeup removers, milk makeup removers, eyeshadows, lipsticks, lip glosses, lip protectors, and powders.

Another aspect of the invention relates to the topical use on the skin of the combination of organic compounds capable of promoting sequential photon transfer between UV light and red light, and precursors of PpIX, to promote physiological stimulation of ROS-dependent tissue, including the activation of proliferation and differentiation programs in tissue stem cell niches and already differentiated cells in the epidermis and dermis, with cosmetic or pharmaceutical applications.

These applications refer to the repair or improvement of the adverse effects on the skin caused by daily stress, sun exposure, or environmental pollutants, as well as premature or natural tissue aging. "Repair or improvement" is understood as stopping, reversing, improving, reducing, and/or diminishing defects, imperfections, or unaesthetic conditions of the skin, including but not limited to fine or deep wrinkles, stretch marks, crow's feet, under-eye circles, hair loss, spider veins, hyperpigmentation, hypopigmentation, discoloration, age spots, loss of skin radiance, freckles, imperfections, tissue fragility, dryness, cracks, tactile roughness, atopic dermatitis, acne, rosacea, psoriasis, and eczema. The repair and improvement of these adverse aspects of the skin rely on stimulating the physiological processes of skin homeostasis and regeneration, which, in turn, are based on the regulated activation of the proliferation and differentiation programs of tissue stem cell niches and other cellular types such as fibroblasts, keratinocytes, Merkel cells, Langerhans cells, and melanocytes. In particular, these applications include the stimulation of cellular production of dermal extracellular matrix components, primarily collagen and elastin, to promote smoothness and firmness of the skin and wrinkle reduction, as well as the stimulation of epidermal stem cell niches to promote skin tone regeneration and maintenance, and the stimulation of hair follicle stem cell niches to prevent hair loss or induce hair growth.

Thus, in a particular aspect, the invention relates to a composition as described above for use as a medicament. Additionally, the invention relates to a composition as described above for use in the treatment of defects, imperfections, or unaesthetic conditions of the skin, including fine or deep wrinkles, stretch marks, crow's feet, under-eye circles, hair loss, spider veins, hyperpigmentation, hypopigmentation, discoloration, age spots, loss of skin radiance, freckles, imperfections, tissue fragility, dryness, cracks, tactile roughness, acne, rosacea, atopic dermatitis, psoriasis, and eczema. Furthermore, the invention also encompasses the use of a composition as described above for the treatment of the effects of solar radiation on human skin, including burns and sunspots.

Another aspect of the invention relates to the topical use on the skin of a combination of organic compounds capable of promoting sequential photon transfer between UV light and red light to enhance tissue photoprotection against UVB/A solar radiation, thereby preventing and/or repairing sunburns and sunspots, as well as other deleterious effects of skin overexposure to solar radiation, such as premature tissue aging or skin cancer.

Thus, in another particular aspect, the invention relates to a non-therapeutic use of a composition as described above as a skin photoprotector. Additionally, the invention relates to a non-therapeutic method for skin photoprotection, which involves topically applying a composition as described above. These aspects of the invention pertain to the cosmetic, non-therapeutic use of the compositions of the present invention as sunscreens in the form of creams, lotions, sprays, and similar formulations, as described above. In the context of the invention, the composition for use as a sunscreen is one with a sun protection factor (SPF) of at least SPF-20, at least SPF-25, at least SPF-30, at least SPF-35, at least SPF-40, at least SPF-45, or at least SPF-50.

The examples provided below demonstrate that the combination of organic compounds such as flavonoids (e.g., morin) and/or cannabinoids (e.g., cannabidiol) capable of absorbing UVB light and emitting blue/green light, and chlorophylls (e.g., chlorophyll A), capable of absorbing blue/green light and emitting red light, in the presence of protoporphyrin IX (PpIX) precursors such as methyl-aminolevulinate (m-ALA), not only protects primary fibroblasts from lethal doses of UVB radiation but also stimulates cell proliferation and differentiation programs and activates the production of extracellular matrix proteins such as collagen and elastin, in a process that is dependent on ROS. Furthermore, it is demonstrated that flavonoids or cannabinoids alone can protect cells in cultures from UVB radiation but cannot stimulate proliferation or extracellular matrix protein synthesis, while chlorophyll alone is neither capable of protecting against UVB radiation nor stimulating cells in any way, thereby de facto implying the existence of a sequential photon transfer (SPT) process that is ultimately coupled to the intracellular production of non-lethal levels of ROS.

All the terms and embodiments described above are applicable to any aspect and embodiment of the invention. In the present invention, the singular terms "the," "a," "an," equally encompass their corresponding plurals unless the context clearly indicates that the term specifically refers to a singular entity. The term "comprises" or "comprising," as used herein, also encompasses "consists of" or "consisting of".

## EXAMPLES

The following invention is described through the following examples, which should be interpreted as merely illustrative.

## Example 1: Sequential Photonic Transfer (SPT) Concept

The present invention proposes the innovative idea of using a Sequential Photonic Transfer (SPT) process between organic/plant-derived compounds, coupled with a transient and stimulating production of ROS in the skin, as a basis for designing dermocosmetic compositions/products that effectively promote broad-spectrum photoprotection and skin regeneration. Thus, the inventors have defined Sequential Photonic Transfer (SPT) as a process that involves the absorption by an organic compound of photons of a certain wavelength, followed by the emission of photons of a different wavelength by this compound, typically longer in the electromagnetic spectrum than the initial photon. The emitted photons can subsequently be absorbed by another adjacent organic compound to emit photons of an even longer wavelength. The chemical structure of each compound involved in the process determines the corresponding ranges of photon absorption and emission. Depending on their chemical characteristics, a specific combination of organic compounds can sequentially couple the absorption and emission of photons of different wavelengths.

To implement this process topically on the skin as a photoprotective and skin regenerating agent, the inventors propose the design and use of combinations of natural plant-derived compounds to capture ultraviolet (UVB/UVA) light photons, which are the most energetic in the visible region of the electromagnetic spectrum, and convert them into red light photons capable of effectively activating ROS production through photo-stimulation of PpIX or its precursors in the skin. Thus, a compound A or combination of compounds A on the skin's surface would be able to absorb UV light photons, emitting blue light photons with a photoprotective effect, and a compound B or combination of compounds B adjacent to compound A on the skin's surface would absorb these blue light photons, emitting red light. Finally, these red light photons would penetrate the skin epithelium to activate a compound C (PpIX or its precursors) to produce ROS levels in the presence of molecular oxygen in the cells, thereby stimulating skin regeneration (Figure 2). Based on their molecular characteristics, the inventors have identified several groups of plant compounds that would fulfill the requirements of the Sequential Photonic Transfer (SPT) process:

- Flavonoids: with absorption peaks in UVA and UVB and emission of blue light photons when excited with UV light.
- Cannabinoids: with absorption peaks in UVA and UVB complementary to those of flavonoids and emission of blue light photons when excited with UV light.
- Chlorophylls, Xanthenes, Carotenes: with absorption peaks in the blue region and emission of red light when excited with blue light photons.

-In vitro Validation of Sequential Photonic Transfer (SPT) Process Coupled with ROS Production.

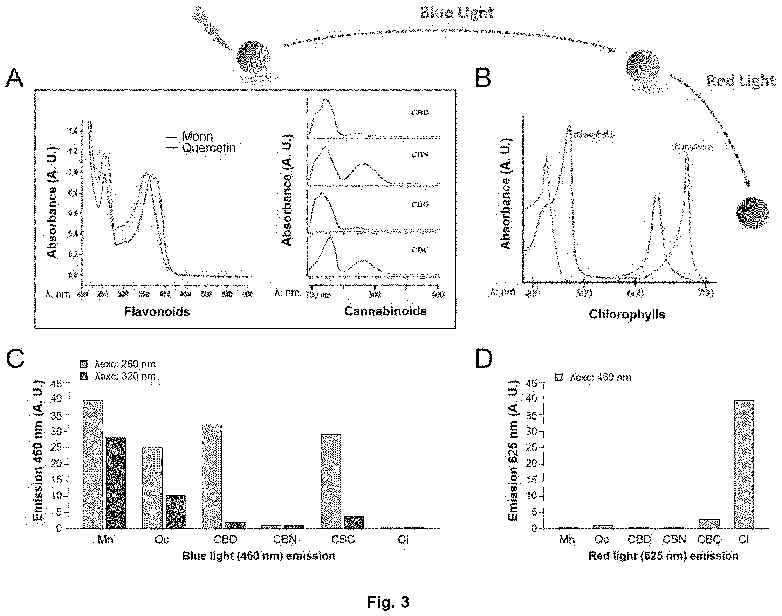

For the in vitro validation of the Sequential Photonic Transfer (SPT) process, different molecules belonging to the groups of plant-derived compounds previously identified as potential targets have been selected. In particular, the selected molecules are morin and quercetin (flavonoids), cannabinol (CBN), cannabidiol (CBD), and cannabichromene (CBC) (cannabinoids), and chlorophyll A, which are fully representative examples of each target compound group. Notably, flavonoids and cannabinoids have complementary absorption spectra in the UVB/A region. Flavonoids typically have absorption peaks around 260 and 380 nm, while cannabinoids have absorption peaks between 230 and 300 nm (Figure 3A). On the other hand, chlorophylls (chlorophyll A and chlorophyll B) have absorption peaks in the blue region of the visible spectrum around 450 nm (Figure 3B). For these compounds, their emission capacity at 460 nm (blue light) when excited by 280 and 320 nm light (UVB/A) and their emission capacity at 625 nm (red light) when excited by 460 nm light have been evaluated using spectrofluorimetry. As expected, the obtained results show that both flavonoids and cannabinoids (except significantly CBN) exhibit high emission of blue light when excited by UVB/A light (Figure 3C), and virtually no emission of red light when excited by blue light (Figure 3D). On the other hand, chlorophyll A shows no emission of blue light when excited by UVB/A light (Figure 3C) but strong emission of red light when excited by blue light (Figure 3D).

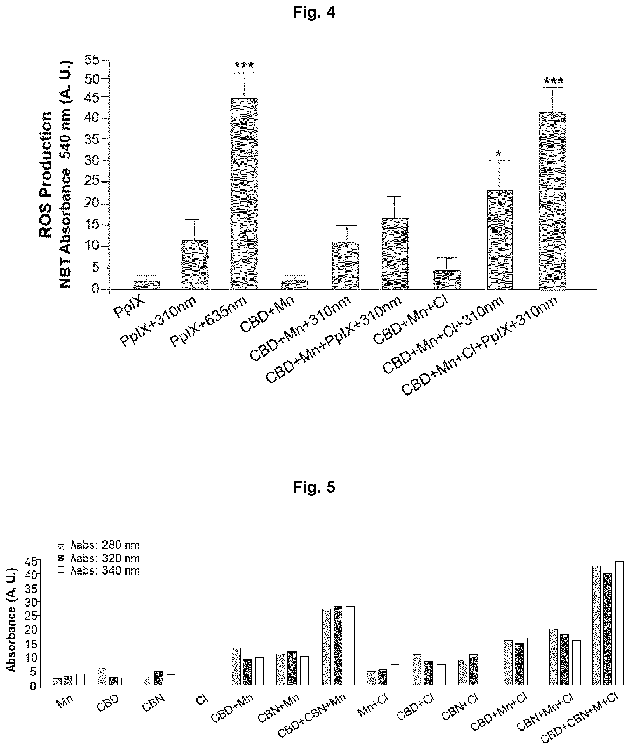

To demonstrate the existence of an SPT process between flavonoids/ cannabinoids (or equivalent molecules) and chlorophylls (or equivalent molecules) coupled with ROS production mediated by PpIX, the oxidation of Nitro Blue Tetrazolium (NBT) in hydroalcoholic solution has been quantified colorimetrically in the presence of different compound combinations. NBT is a specific molecular sensor for superoxide anion production, which is the first type of ROS produced during the reduction of molecular oxygen to generate water. In the presence of superoxide anion, NBT, a colorless molecule in its reduced state, oxidizes to form a chromophore with an absorption peak at 540 nm. The degree of oxidation, and therefore the absorbance at 540 nm, is directly proportional to ROS production. As shown in Figure 4, in a hydroalcoholic solution of PpIX under basal conditions or excited with UV light (310 nm), no significant ROS production is detected; however, if this solution is excited with red light (635 nm), capable of photo-stimulating the redox activity of PpIX to produce superoxide anion from molecular oxygen, NBT oxidation is greatly increased, as expected (Figure 4). Importantly, UV excitation (310 nm) of solutions containing flavonoids and cannabinoids alone or in combination with PpIX does not induce significant ROS production, indicating that the blue light production from these compounds when excited by UV light is not able to directly stimulate ROS production from molecular oxygen or using PpIX as an intermediary (Figure 4). Likewise, chlorophyll A in a UV-excited solution is also unable to induce significant NBT oxidation (Figure 4). In the same vein, the combination of flavonoids and cannabinoids with chlorophyll A under basal conditions does not induce significant ROS production. However, the combination of flavonoids and cannabinoids with chlorophyll A excited with UV light (310 nm) induces significant NBT oxidation, indicating that the conversion of UV light photons into red light alone is capable of stimulating ROS production to some extent in vitro, probably due to the light absorption peak of molecular oxygen in the red/infrared region that could be sufficient to directly stimulate ROS production (Figure 4). However, the combination of flavonoids/cannabinoids, chlorophyll A, and PpIX excited with UV light (310 nm) induces a potent and significant ROS production equivalent to that obtained by exciting PpIX with red light (Figure 4).

Overall, these results demonstrate the existence of an SPT process between flavonoids/cannabinoids and chlorophyll A that can convert UV light photons into red/infrared light photons. These results also demonstrate that this SPT process can be coupled to ROS production either directly or enhanced by the presence of an appropriate photosensitizer, in this case, PpIX.

-Evaluation of the UVB/A absorption capacity of different combinations of organic compounds capable of promoting a SPT process.

The absorbance of various organic compound mixtures, including flavonoids, cannabinoids, and chlorophylls, was quantified in alcoholic solutions to assess their ability to absorb UVB/A radiation (280, 320, and 340 nm). The results obtained indicate that, as expected, based on their spectroscopic characteristics (see Figure 3), a combination of flavonoids and cannabinoids exhibits a notable capacity to absorb UVB/A radiation, which is significantly enhanced by the presence of chlorophyll A (Figure 5). These results suggest that, based on the absorbance values obtained at different wavelengths in the UVB/A region, the in vitro and/or in vivo determination of the Sun Protection Factor (SPF) will show values ranging from 20 to 35 for the combinations of flavonoids/cannabinoids or flavonoids/cannabinoids/chlorophylls.

**Example 2: Validation of the efficiency of the SPT process in promoting UV photoprotection and cutaneous regeneration in a first experimental skin model**

To validate in vitro the capacity of the SPT process coupled with ROS production as a mechanism capable not only of protecting mammalian cells and tissues, particularly the skin, against UVB/A radiation but also of using this UVB/A radiation as a catalyst to enhance cell proliferation and tissue regeneration, primary human skin fibroblast cultures have been used as an experimental model.

## 2.1 Materials and Methods

• Experimental Model

For the development of the assays carried out in this project, primary cultures of human fibroblasts obtained from skin biopsies of healthy volunteers and regions of healthy skin from young patients (neonates to 13 years old) were used as an in vitro model. Skin biopsies were given by the Dermatology Unit at the Ramón y Cajal Hospital, with the consent of patients and the approval by the Ethics Committee of the Ramón y Cajal Hospital. The biopsies were processed by mechanical disaggregation using a scalpel and enzymatic digestion with Collagenase A (1 mg/ml, Roche) for collagen digestion and cell dissociation to obtain fibroblasts.

• Cultivation and Maintenance of Human Fibroblast Cell Lines

The cell line was cultured following standard protocols (Ng W, Ikeda S. "Standardized, Defined Serum-Free Culture of a Human Skin Equivalent on Fibroblast-Populated Collagen Scaffold." Acta Derm Venereol. 91:387-91, 2011). The cell line was grown in Dulbecco's Modified Eagle's Medium (DMEM) without pyruvate (Gibco), supplemented with 2 mM L-glutamine (Gibco), 10% fetal bovine serum (FBS) (Gibco), and 1X diluted antibiotic-antimycotic (Gibco). All cultures were maintained at 37°C in a humid atmosphere with 5% $CO_2$. Pyruvate-free culture medium was used for the subsequent analysis of the release of the cytoplasmic lactate dehydrogenase (LDH) enzyme from dead and/or lysed cells, which catalyzes the oxidation of lactate to pyruvate, as described in section 3.10 of materials and methods.

For cell line maintenance, as well as for the various treatments carried out, 100 cm2 surface Petri dishes (P100, Falcon) and 6-well plates (MW6, Falcon), respectively, were used and treated for cell adhesion. Cells were maintained at a confluence of 20-90%, and the culture medium was renewed every other day and subcultures were performed (when reaching 70-80% confluence) by cell dissociation using Trypsin-EDTA (Sigma-Aldrich), depending on the doubling time of each cell line. In addition, cells were also seeded on 12 mm diameter round glass coverslips (Superior Marienfeld) placed in the 6-well plates for subsequent protein immunolocalization analysis.

• Ultraviolet B light damage assay in primary cultures of human fibroblasts.

To determine the time required for the detection of cellular damage after ultraviolet B light irradiation, 700,000 human fibroblasts were seeded per well in 6-well plates (MW6, Falcon) on glass coverslips. After seeding, the cell cultures were maintained in complete medium for 24 hours until reaching 80-90% confluence. After this time, the cells were irradiated for 5 minutes with a 311 nm wave ultraviolet B light source (Dermalight 200, Dr Hönle Medizintechnik) positioned on top of the plate and kept 2 cm away from the cell cultures. Subsequently, the cultures were maintained in complete DMEM culture medium for the following established times: 10 minutes, 30 minutes, 1 hour, 2 hours, 4 hours, 16 hours, 24 hours, and 48 hours. Negative controls without UVB light were also included. After the designated time, the cells were fixed with 3.7% formaldehyde in PBS for subsequent protein immunolocalization analysis.

• Treatment with plant chemicals (Chlorophyll A and Morin or Cannabidiol) and methyl aminolevulinate (m-ALA) as a precursor of the endogenous photosensitizer Protoporphyrin IX (PpIX) in human fibroblasts.

To carry out the different treatments, the same number of cells (700,000) were seeded in 6-well plates (MW6, Falcon). After seeding, the cell cultures were maintained in complete medium for 24 hours until reaching 80-90% confluence. After this time, the complete medium was removed, and the cell cultures were incubated with a combination of 1.6 mM Morin (Sigma) or 1 mM Cannabidiol and 1.11 mM Chlorophyll A (Sigma), and 0.1 mM m-ALA in complete DMEM medium without FBS for 4 hours in darkness at 37°C in a humid atmosphere with 5% $CO_2$. Subsequently, the culture plates were irradiated with a 311 nm wavelength ultraviolet B light source (Dermalight 200, Dr Hönle Medizintechnik) for 5 minutes. Negative controls without m-ALA, chlorophyll, morin, cannabidiol, and light were included. After completing the procedure, the culture medium was removed, the cells were washed with phosphate-buffered saline (PBS), and they were maintained in fresh complete DMEM medium for subsequent analysis. All plates were cultured until the collection of cell samples at 4, 24, and 48 hours for different assays.

**• Immunolocalization of γH2AX and Ki67 proteins in the cell lines used**

For protein immunolocalization in human fibroblasts grown on glass coverslips, the cells were fixed with 3.7% formaldehyde in PBS for 20 minutes at room temperature (RT) 4, 24, and 48 hours after the treatments described in section 3.4. Subsequently, the coverslips were washed with PBS, and 0.5% Triton X-100 (Sigma-Aldrich) in PBS was added for 30 minutes to permeabilize the membranes of the fixed cells. After permeabilization, nonspecific binding sites were blocked with 0.5% PBS-BSA (bovine serum albumin diluted in PBS) for 30 minutes at RT. Then, the samples were incubated with the corresponding primary antibody (rabbit anti-Ki67 and mouse anti-yH2AX) diluted 1:100 in PBS-BSA overnight at 4°C in a humid chamber. After washing the samples with PBS, the coverslips were incubated with the corresponding secondary antibody (Alexa FluorTM 546 goat anti-rabbit IgG (H+L) or Alexa FluorTM 488 goat anti-mouse IgG (H+L), Life Technologies) at a dilution of 1:200 in PBS-BSA and DAPI (5 ng/ml, Sigma-Aldrich) for nuclear counterstaining for 1 hour at RT in darkness. Finally, the samples were mounted with Prolong aqueous mounting medium (Invitrogen). The samples were observed using a Nikon Eclipse Ci-L 100-240V 0.2A 50/60Hz fluorescence microscope coupled with a CCD camera Progress (Gryphax) by exciting the corresponding fluorochrome.

**• Viability assay in primary fibroblast culture using crystal violet staining**

To determine cell viability and quantify cell death after the different treatments, cells were stained with crystal violet (Feoktistova, M., Geserick, P., & Leverkus, M. (2016). Crystal Violet Assay for Determining Viability of Cultured Cells. Cold Spring Harbor Protocols, 2016(4), pdb.prot087379). After removing the culture medium in which the cells had been grown, they were fixed at 4, 24, and 48 hours with cold methanol (-20°C) for 10 minutes at RT. Subsequently, the cells were stained with 0.1% crystal violet for 40 minutes at RT. Then, after removing the crystal violet and rinsing off the excess with running water, the plates were incubated in a 37°C incubator for 1 hour. Finally, to dissolve the stain, 10% acetic acid was added and kept on agitation for 15 minutes at RT. The absorbance was measured in 96-well plates at 620 nm using the Tecan's Sunrise absorbance microplate reader.

**• Viability assay and cell cycle analysis in primary human fibroblast culture using flow cytometry.**

To determine cell viability and the cell cycle phase of primary human fibroblasts after different treatments at 4, 24, and 48 hours, propidium iodide (PI) staining was performed. PI is a fluorogenic compound that binds stoichiometrically to nucleic acids, and its fluorescence is proportional to the DNA and RNA content of a cell, decreasing when cells undergo apoptosis (Riccardi, C., & Nicoletti, I. (2006). Analysis of apoptosis by propidium iodide staining and flow cytometry. Nature Protocols, 1(3), 1458-1461). To perform the staining, cell supernatants along with the collected cell suspension after trypsinization were centrifuged at 1500 rpm for 10 minutes, and the resulting cell pellets were fixed with cold 70% ethanol for 16 hours at -20°C. Subsequently, the samples were centrifuged at 200 g for 10 minutes at 4°C and, after washing with cold PBS, they were resuspended in a PBS solution containing the following staining mixture: propidium iodide (1 mg/ml), RNase free of DNases (10 μg/mL), and 0.1% Triton (v/v) in PBS. Finally, the samples were transferred to 1.5 mL Eppendorf tubes and incubated at 37°C for 15 minutes. The final samples were stored at 4°C until flow cytometry analysis (Flow Cytometry Service, UAM).

**• RNA extraction and gene expression analysis**

To determine the mRNA expression levels in the cell pellets of human fibroblasts collected by trypsinization at 4, 24, and 48 hours after the treatments, RNA extraction and purification were performed using the RNeasy Mini Kit (Qiagen) following the manufacturer's instructions. The concentration and purity of mRNA were quantified by determining its absorbance using a drop spectrophotometer (SimpliNanoTM), and RT-PCR was carried out to generate cDNA using the FastGene Scriptase Basic cDNA Synthesis Kit (NIPPON Genetics EUROPE).

**• Collagen Iα1 Human concentration quantification in response to treatment**

To study the release of human collagen Iα1 in the culture medium, a commercial kit, Human Pro-Collagen I alpha 1 DuoSet ELISA, 5 Plate, was used following the manufacturer's instructions, using for this purpose the supernatants obtained from the culture media of the different experimental conditions established in the treatment. The supernatants were exposed to a mouse Anti-Human Pro-Collagen Iα1 capture antibody and decreasing concentrations of Recombinant Human Pro-Collagen Iα1 Standard for subsequent analysis of the concentrations obtained using a standard curve. The samples were then incubated for 2 hours at room temperature in the dark with a detection antibody, followed by the addition of Streptavidin-HRP for 20 minutes in the dark. Optical density determination was performed using the Tecan's Sunrise absorbance microplate reader at 450 nm and 570 nm. After obtaining the optical densities, the absorbance at 450 nm and 570 nm were subtracted, and a standard curve was generated to interpolate the data obtained from the different conditions.

• Determination of cellular cytotoxicity by quantifying the concentration of the lactate dehydrogenase (LDH) enzyme released into the medium after treatment

To assess cellular cytotoxicity, the concentration of lactate dehydrogenase (LDH) enzyme, that catalyses the pyruvate conversion by reducing NAD+ to NADH, released into the cell culture medium from dead cells was determined using the CyQUANT™ LDH Cytotoxicity Assay Kit (Invitrogen) following the manufacturer's instructions and triplicating the treatments. The assay is based on a colorimetric method involving the reduction of tetrazolium salt (INT) to form red formazan crystals, which is proportional to the amount of LDH released in the medium and indicative of cytotoxicity. Absorbance was measured using the Tecan's Sunrise absorbance microplate reader at 680 nm and 490 nm. After obtaining the corresponding optical densities, the absorbance at 490 nm was subtracted from the absorbance at 680 nm to obtain the percentage of cytotoxicity for each sample using the following formula:

$$\% \text{ Cytotoxicity} = \left[ \frac{\text{Compound-treated LDH activity} - \text{Spontaneous LDH activity}}{\text{Maximum LDH activity} - \text{Spontaneous LDH activity}} \right] \times 100$$

Formula for calculating the percentage of LDH-associated cytotoxicity.

• Statistical Methods

For all the experimental designs, a minimum number of samples ($n \geq 3$) were included to obtain statistical significance in the control and treatment conditions, performing a comparison of the variables studied between control and treatment groups. All data obtained from different measurements and analyses were entered into an Excel file, and the independent values of each treatment were normalized to their respective controls, establishing ratios and comparing them using the Student's t-test for metric or non-metric samples based on the analysis of variance performed by the Fisher's t-test. Subsequently, the values were represented in bar graphs. For the statistical analysis of gene expression data obtained by PCR, the data were entered into an Excel file and analyzed using the comparative Ct method, representing the changes in gene expression as 2-$\Delta$Ct values, and normalizing the expression to the control gene used (18s). The results were then presented in bar graphs.

## 2.2 Results

-Cell damage caused by UVB light irradiation in primary cultures of human fibroblasts begins to manifest at 24 hours after exposure.

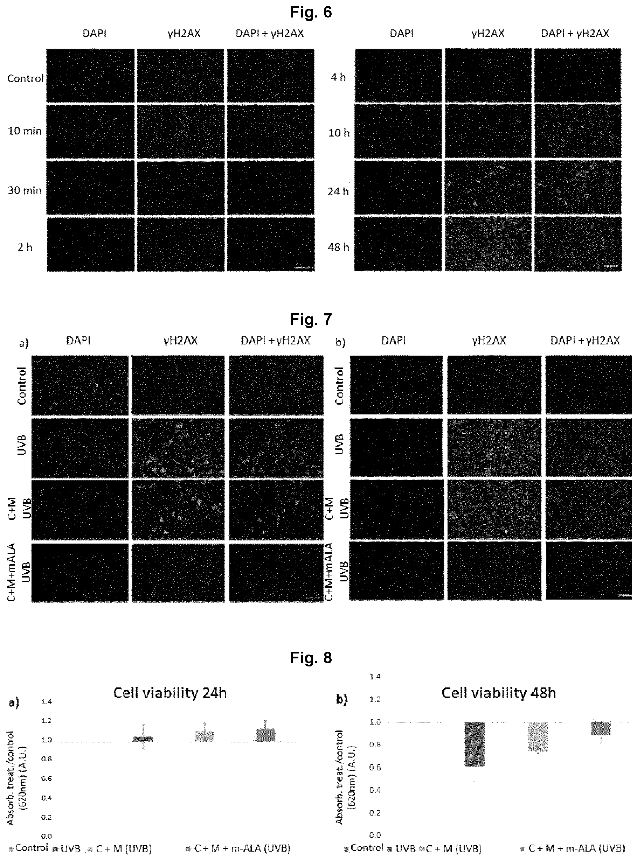

Firstly, to determine the time at which cellular damage induced by 5-minute UVB light irradiation manifests in primary cultures of human fibroblasts, the marker for double-strand DNA breaks, histone yH2AX, commonly used as a positive control for UV-induced damage (Yuan, J., Adamski, R., & Chen, J. (2010). Focus on histone variant H2AX: to be or not to be. FEBS letters, 584(17), 3717-3724), was analyzed using immunodetection at different established times (10 minutes, 30 minutes, 1 hour, 4 hours, 16 hours, 24 hours, and 48 hours). The results obtained, as shown in Figure 6, indicate that genomic damage following UVB induction begins to manifest with the expression of the γH2AX marker at 24 hours, and this expression is maintained at 48 hours; therefore, both time points were selected for the assays conducted in this study.

-Reduction of damage and cell death against UVB radiation in primary cultures of human fibroblasts treated with the combination of natural extracts (chlorophyll A and morin) and m-ALA

To analyze the protective role of the combination of natural extracts (chlorophyll A and morin), along with the precursor of the endogenous photosensitizer PPIX (m-ALA), against UVB radiation, primary human fibroblast cell cultures were incubated with the different treatments described in the materials and methods section and subjected to UVB irradiation. After 24 and 48 hours, the cultures were fixed. Next, to confirm the observed reduction in cell death and determine the potential protective role of the complete treatment against UVB-induced damage in primary cultures of human fibroblasts, immunodetection of the damage histone yH2AX was performed under the different conditions studied. As shown in Figure 7, the control group (non-irradiated and untreated) did not show expression of the γH2AX histone at any of the studied time points. However, UVB light irradiation, as described, induced expression of the damage histone yH2AX, which notably decreased in cells treated with the combination of chlorophyll A and morin at 48 hours after UVB irradiation. As expected, cells treated with the combined treatment of morin, chlorophyll A, and m-ALA did not show expression of yH2AX in response to UVB radiation, suggesting a clear protective effect of this complete treatment against damage caused by UVB irradiation.

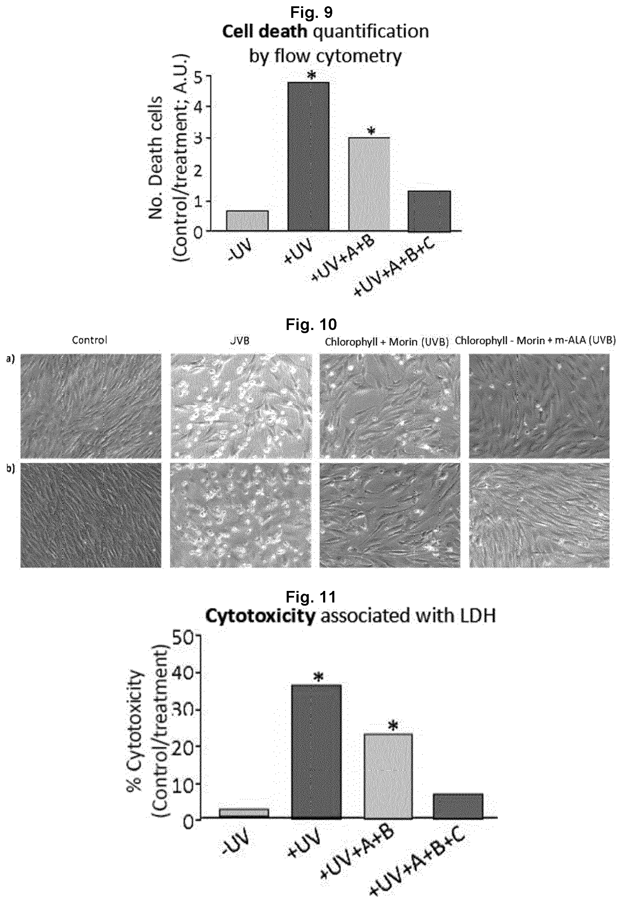

To assess cell viability, the cultures were fixed and stained with crystal violet after 24 and 48 hours of UVB irradiation. Cell viability was quantified by measuring absorbance at 620 nm and represented as a percentage, assuming 100% viability in the control condition. Figure 8 shows that treatment with natural extracts, as well as the combination of these extracts with the precursor of protoporphyrin IX, increases cell viability compared to cells irradiated with UVB light alone at 24 hours after treatment, this increase in viability being significant at 48 hours after treatment. To confirm the results of cell viability, 24 and 48 hours after treatments, cell cycle analysis was performed using flow cytometry with propidium iodide staining, which penetrates the membrane of damaged cells and intercalates between the two DNA strands, staining cells that have undergone cell death. Figure 9 shows that, as expected, based on the previous result, there is a significant increase in cell death in cells irradiated with UVB light alone at both 24 and 48 hours after irradiation, with a slight decrease in cell death in the presence of the combination of chlorophyll A and morin at 48 hours after treatment. However, as anticipated, the complete treatment leads to a drastic reduction in cell death in response to UVB irradiation at both 24 and 48 hours, with cell death levels similar to control cells, thus demonstrating a clear protective effect of the complete treatment against damage caused by UVB irradiation.

Additionally, to corroborate these results, bright-field images of the cultures were taken using an inverted microscope regularly until the control cells reached confluence (48 hours). Figure 10 shows that, as expected, based on the previous results, cell death was observed 24 hours after irradiation of cell cultures with UVB light, mainly manifested as cellular debris in the supernatant, and it was nearly complete at 48 hours. Partial reduction of cell death was observed in cells treated with natural extracts, with a more noticeable effect in the case of the combined treatment of natural extracts with the precursor of PpIX (m-ALA) at both 24 and 48 hours after treatment.

Finally, based on the results obtained regarding the decrease in yH2AX histone expression after incubation of primary human fibroblast cultures with the combined treatment of natural extracts (with and without m-ALA), indicative of reduced cellular damage, and the observed decrease in cell death, the release of lactate dehydrogenase (LDH) enzyme into the cell culture medium was analyzed in all conditions, as LDH is released when the plasma membrane ruptures during cell death (Ana Celeste Ximenes Oliveira, Ismael Ángel Rodriguez, Ingrid Garzón, Miguel Alaminos. Estudio de viabilidad celular en un modelo experimental de fibroblastos gingivales humanos cultivados en ausencia de suero bovino fetal, Actual. Med. Vol. 95 (2010) n°780, May/August 2010, pags. 013 - 018). LDH release and the percentage of cytotoxicity were determined using colorimetric quantification and subsequent optical density measurement as described in the materials and methods section. Based on this, Figure 11 shows that cytotoxicity associated with UVB irradiation increases significantly compared to the control, however, in samples treated with chlorophyll A and morin, there is a clear decrease in cytotoxicity at 24 hours after treatment and UVB irradiation. This protective effect is further intensified in cells subjected to the combined treatment of natural extracts with m-ALA at 48 hours after treatment and UVB irradiation of the cell cultures.

-Increase in S-phase and cell proliferation in primary human fibroblast cultures in response to the complete treatment with chlorophyll A, morin, and m-ALA as a precursor of PpIX

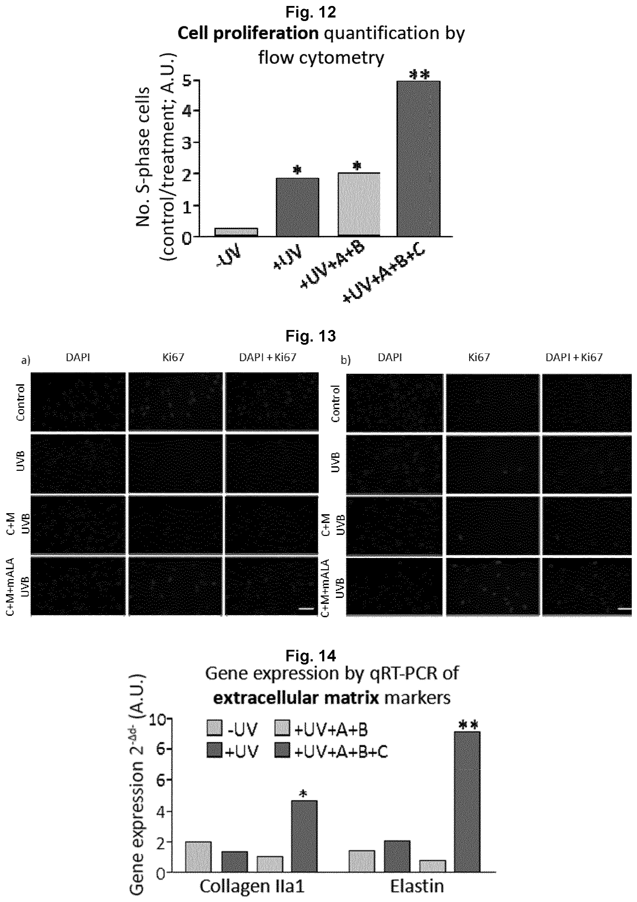

Considering that the complete treatment involves the use of m-ALA, which is incorporated into the biosynthetic pathway of heme groups, resulting in the formation of the endogenous photosensitizer PpIX, and that its irradiation with appropriate wavelength light (635 nm) leads to the generation of non-lethal doses of ROS, which induce an increase in cell proliferation (Carrasco, E., Blázquez-Castro, A., Calvo, M. I., Juarranz, Á., & Espada, J. (2016). Switching on a transient endogenous ROS production in mammalian cells and tissues. Methods (San Diego, Calif.), 109, 180-189), it was proposed to analyze whether the generation of an electron chain from UVB light (311 nm), after being absorbed by the different plant extracts, first morin and then chlorophyll A, would stimulate the absorption peak of PpIX (635 nm), which upon excitation would lead to the generation of a transient and sublethal dose of ROS. In this regard, we wanted to investigate whether a potential increase in the S-phase of cell cycle (DNA synthesis) was occurring after UVB irradiation in primary human fibroblast cultures after the concurrent treatment of plant extracts with m-ALA. Thus, primary human fibroblast cultures were incubated with the complete treatment of chlorophyll A, morin, and m-ALA for 4 hours, followed by UVB light irradiation. At 24 and 48 hours after treatment, the obtained pellets and supernatants were stained with propidium iodide and analyzed by flow cytometry. Figure 12 shows that, as expected, an increase in the S-phase of cell cycle is observed at 24 and 48 hours after treatment of the cell cultures with chlorophyll A and morin, with and without m-ALA, and subsequent UVB irradiation, this increase being particularly significant at 48 hours after concurrent treatment of the plant extracts with m-ALA. Additionally, as described in the literature, an increase in the S-phase is also observed in cell cultures solely irradiated with UVB (Imray, P., Mangan, T., Saul, A., & Kidson, C. (1983). Effects of ultraviolet irradiation on the cell cycle in normal and UV-sensitive cell lines, with reference to the nature of the defect in xeroderma pigmentosum variant. Mutation research, 112(5), 301-309), with the highest increase observed at 24 hours after irradiation.

Once the increase in the S-phase in the cell cultures in response to the treatment was determined, it was desired to confirm an increase in cell proliferation by studying the expression of the proliferation marker Ki67 in primary human fibroblast cultures through immunodetection. Figure 13 shows that, in line with the results obtained from the flow cytometry assay, the presence of the proliferation marker Ki67 increases in fibroblasts incubated with the complete treatment containing m-ALA compared to cells solely irradiated with UVB light, while remaining similar to the control (non-irradiated and untreated) in cells treated with chlorophyll A, morin without m-ALA. Additionally, the presence of the Ki67 marker decreases in fibroblast cultures solely irradiated with UVB and does not increase in those treated with chlorophyll A and morin.

-Increase in extracellular matrix components in primary human fibroblast cultures with the complete treatment combining chlorophyll A and morin with m-ALA as a precursor of protoporphyrin IX

Considering that UVB radiation induces damage to dermal fibroblasts as a result of elevated ROS generation, leading to a decrease in ECM production and remodeling associated with collagen decrease (Rittié, L., & Fisher, G. J. (2015). Natural and sun-induced aging of human skin. Cold Spring Harbor perspectives in medicine, 5(1), a015370), and that in previous unpublished laboratory experiments, an increase in collagen fibers in the dermis of young mice had been determined as a result of fibroblast proliferation following the transient and localized generation of non-lethal levels of ROS through TF, it was desired to analyze if the proposed treatment was effective in preventing UVB-induced damage to collagen fibers and if it also stimulated their production associated with increased fibroblast proliferation. For this purpose, quantification of the concentration of human collagen I$\alpha$1 released into the culture medium 24 hours after the treatments was carried out, analyzed by Enzyme-Linked Immunosorbent Assay (ELISA). A decrease in the deposition of Collagen I$\alpha$1 was observed in fibroblasts irradiated with UVB compared to the control. However, cells exposed to the treatment with chlorophyll A and morin irradiated with UVB showed similar deposition to the control cells, with a slight increase in deposition observed in fibroblasts treated with the combination of chlorophyll A, morin, and m-ALA 24 hours after treatment administration.

Finally, to corroborate this result, validation was performed using quantitative PCR of several extracellular matrix markers, such as Collagen III (Col IIIA1), Collagen II (Col IIA1), and Elastin (ELN), using cDNA obtained from samples of primary human fibroblast cultures exposed to the different established conditions 24 and 48 hours after treatments and UVB irradiation, with their expression normalized to 18s ribosomal RNA. The results obtained are shown in Figure 14, demonstrating a significant increase in the expression of extracellular matrix markers Col IIIA1, Col IIA1, and ELN in human fibroblasts exposed to the complete treatment compared to the control and other established conditions, corroborating a protective role of chlorophyll A and morin against UVB light-induced damage, as well as a stimulating role in proliferation when fibroblasts are treated with the complete treatment and irradiated with UVB light.

Example 3: Validation in a second experimental skin model to assess the efficiency of TFS process in promoting UV photoprotection and cutaneous regeneration

Organotypic cultures of human skin, particularly in their early establishment stages, constitute an experimental model that closely recapitulates the molecular and cellular responses of in vivo skin tissue to different stimuli or exposure to exogenous/xenobiotic factors. It is the preferred model recommended by regulatory agencies such as EMA, FDA, and other international organizations to validate and evaluate the efficacy of drugs and cosmetics.

-Maintenance of epidermal integrity and collagen fibers following UVB light irradiation in organotypic cultures of human skin treated with various combinations of natural extracts CBD, morin, and/or chlorophyll A, along with m-ALA

To address the second objective and analyze whether incubation of organotypic cultures of human skin (HSOC, human skin organ culture) with the compositions described in the present invention provided protection against damages caused by UVB irradiation through the TFS phenomenon, HSOC cultures were first established using the standard technique in the field of the invention (Mendoza-Garcia, J., Sebastian, A., Alonso-Rasgado, T., & Bayat, A. (2015). Optimization of an ex vivo wound healing model in the adult human skin: Functional evaluation using photodynamic therapy. Wound repair and regeneration: official publication of the Wound Healing Society [and] the European Tissue Repair Society, 23(5), 685-702) and were incubated with different combinations of natural extracts cannabidiol (CBD) and/or morin and chlorophyll A. After 24 hours of incubation with these phytochemicals, Metvix® (methyl aminolevulinate) was applied to the epidermis of HSOCs as a precursor of PpIX for 2 hours prior to UVB irradiation. To determine the UVB dose at which structural damages occur in untreated skin, an irradiation range between 6 and 12 J, corresponding to an exposure time range between 5 and 10 minutes, was chosen based on literature (Masuma, R., Kashima, S., Kurasaki, M., & Okuno, T. (2013). Effects of UV wavelength on cell damages caused by UV irradiation in PC12 cells. Journal of photochemistry and photobiology. B, Biology, 125, 202-208). Firstly, the maintenance of skin integrity 7 days after irradiation with both UVB doses was analyzed through H&E staining of histological sections of HSOCs treated with the aforementioned combinations of phytochemicals in the presence of m-ALA and compared to the corresponding controls.

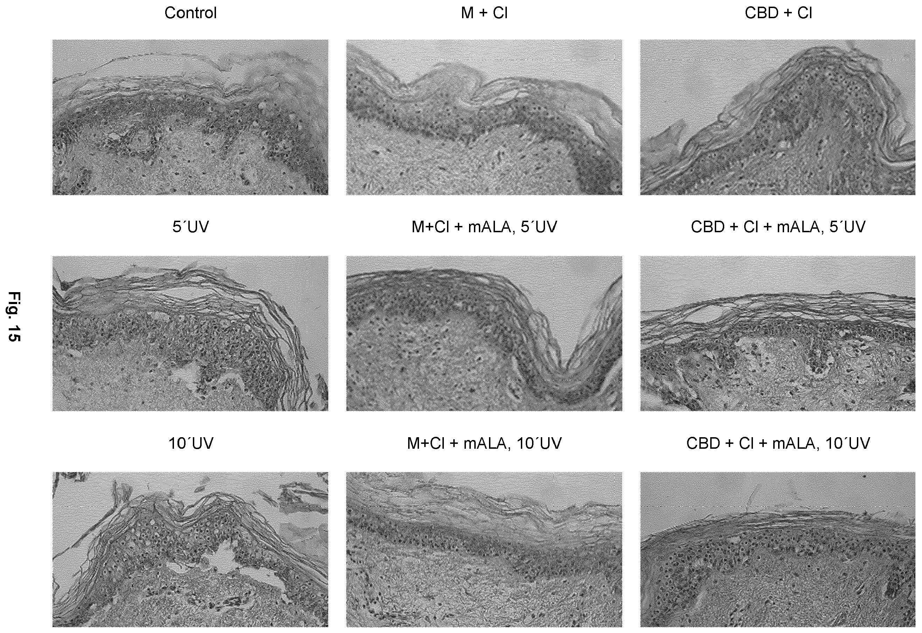

As expected from our previous results, Figure 15 reveals that skin samples irradiated with UVB light exhibited evident dermo-epidermal separation with a discontinuous pattern associated with epidermal ridges and the presence of dermal edema even at 6 J of irradiation, which increased proportionally after 12 J of irradiation, the latter dose also causing alteration of the typical cuboidal morphology of basal layer keratinocytes. However, pre-treatment with combinations of morin, chlorophyll, and m-ALA prevented the appearance of these histological alterations after 6 J of UVB irradiation, maintaining skin integrity similar to the control, and partially after 12 J of UVB irradiation (Figure 16). On the other hand, treatment with a combination of CBD, chlorophyll, and m-ALA partially prevented the appearance of these histological alterations after both 6 J and 12 J of UVB irradiation (Figure 16).

Simultaneously, the effect of incubating the organotypic cultures with different combinations of phytochemicals in the absence of irradiation was analyzed to rule out any potential toxicity. As shown in Figure 16, treatment with the various combinations of phytochemicals in the absence of UVB irradiation did not induce structural alterations in the organotypic cultures of skin, as expected, based on previous laboratory results.

## DESCRIPTION OF THE FIGURES

**Figure 1.** A) Schematic representation of the photodynamic therapy procedure using three elements, a photosensitive agent (PS), light of an appropriate wavelength that can be absorbed by the PS (red arrow), and molecular oxygen to produce reactive oxygen species (ROS). B) Biosynthetic pathway for the endogenous photosensitizer PpIX production in the mitochondria.

**Figure 2.** Schematic representation of the sequential photon transfer process in the skin.

**Figure 3.** Continuous absorption spectrum of representative flavonoids and cannabinoids in the UV region of the electromagnetic spectrum (200-400 nm) (A) and of chlorophylls A and B in the blue-infrared region of the electromagnetic spectrum (400-700 nm) (B). Spectrofluorimetric quantification in hydroalcoholic standard solutions of emission at 460 nm (blue region of the electromagnetic spectrum) of different compounds after excitation at 280 or 320 nm (UVB/A) (C) and of emission at 625 nm (red region of the electromagnetic spectrum) after excitation at 460 nm (D) (Mn: Morin; Qc: Quercetin; CBD: cannabidiol; CBN: cannabinol; CBC: cannabichromene; Cl: chlorophyll A).

**Figure 4.** Quantification of in vitro ROS production measured as absorbance at 540 nm of Nitro Blue Tetrazolium (NBT) compound oxidized by singlet oxygen production in hydroalcoholic solutions of different compound combinations under basal conditions or excited with UV (310 nm) or red (635 nm) light. Results are expressed as mean +/- standard deviation of three independent experiments. *, $P \leq 0.5$; ***, $P \leq 0.01$ (PpIX: Protoporphyrin IX; Mn: Morin; CBD: cannabidiol; Cl: chlorophyll A).

**Figure 5.** Quantification in hydroalcoholic solutions of absorbance at different wavelengths in the UVB/A region (280, 320, and 340 nm) of different organic compound combinations (Mn: Morin; CBD: cannabidiol; CBN: cannabinol; Cl: chlorophyll A).

**Figure 6.** Cellular damage in primary human fibroblast cultures after exposure to UVB light begins to manifest at 24 hours post-exposure. Immunodetection using fluorescence microscopy of the histone $\gamma$H2AX damage marker (green) at different established time points (10 min, 30 min, 1 hour, 4 hours, 16 hours, 24 hours, and 48 hours) in primary human fibroblast culture. Nuclei are counterstained with DAPI (blue). Representative images from 3 experimental replicates. Scale bar = 100 $\mu$m.

**Figure 7.** Cellular damage caused by UVB radiation notably decreases in the presence of the complete treatment with chlorophyll A, morin, and m-ALA in primary human fibroblast cultures. Representative fluorescence microscopy images showing immunodetection of the cellular damage marker $\gamma$H2AX (green) at 24h (a) and 48h (b) after the treatments. Nuclei counterstained with DAPI are shown in blue. Scale bar = 100 $\mu$m. The images are representative of n=3. Equivalent results were obtained using cannabidiol instead of morin.

**Figure 8.** Cell viability increases in primary human fibroblast cultures treated with chlorophyll A (C), morin (M), and the precursor of protoporphyrin IX (m-ALA) compared to cells solely irradiated with UVB light at 24h post-treatment, with a significant increase observed at 48h. Cell viability quantification by crystal violet staining (absorbance of treatment/control measured at 620 nm wavelength; A.U.: arbitrary units) in primary human fibroblast cultures at 24 hours (a) and 48 hours (b) after treatment. *, significant, $p \leq 0.1$. n=3. Error bars indicate standard error. Equivalent results were obtained using cannabidiol instead of morin.

**Figure 9.** Drastic reduction of cell death in primary human fibroblast cultures at 48h after administration of the combination of morin or cannabidiol (A), chlorophyll A (B), with and without m-ALA, irradiated with UVB compared to cells solely irradiated with UVB. Cell death quantification measured by flow cytometry in human fibroblasts previously stained with propidium iodide and expressed as a ratio relative to the control (non-irradiated and untreated). Error bars indicate standard error. *, significant, $p \leq 0.1$. n=3.

**Figure 10.** The complete treatment with chlorophyll A, morin, and m-ALA protects against UVB-induced damage in primary human fibroblast cultures. Representative bright-field images taken with an inverted microscope at 24h (a) and 48h (b) after treatment administration. Representative images from 3 experiments. Scale bar = 100 $\mu$m. Equivalent results were obtained using cannabidiol instead of morin.

**Figure 11.** Decreased cytotoxicity associated with LDH release as a result of the combined treatment of morin or cannabidiol (A), chlorophyll A (B), and m-ALA (C) in UVB-irradiated primary human fibroblast cultures at 48h post-treatment. Quantification of lactate dehydrogenase (LDH) enzyme release into the cell culture medium 48h after treatment administration and UVB light irradiation, expressed as a percentage of cytotoxicity using the formula: % cytotoxicity = (treatment-control total) / (maximum LDH release-control total) x 100. n=2.

**Figure 12.** Significant increase in the S phase of cell cycle in UVB light-irradiated primary human fibroblast cultures in the presence or absence of the combination of morin or cannabidiol (A), chlorophyll A (B), with and without m-ALA (C) compared to the control (non-irradiated and untreated). Cell cycle S phase quantification relative to the control at 48h after treatment administration using propidium iodide staining and subsequent flow cytometry analysis. Error bars indicate standard error. *, significant, $p \leq 0.1$. n=3.

**Figure 13.** Cell proliferation increases in primary fibroblast cultures in the presence of the complete treatment with morin, chlorophyll A, morin, and m-ALA. Immunodetection of the proliferation marker Ki67 using fluorescence microscopy in primary human fibroblast cultures at 24h (a) and 48h (b) after treatment administration. Scale bar = 100 $\mu$m. The images are representative of 3 experiments. Equivalent results were obtained using cannabidiol instead of morin.

**Figure 14.** Increase in extracellular matrix markers in primary human fibroblast cultures at 24h after the complete treatment with morin or cannabidiol (A), chlorophyll A (B), and m-ALA, and UVB irradiation compared to the control and the rest of the established conditions. Quantification of the expression of extracellular matrix genes Collagen IIA1 and Elastin using quantitative PCR. Ribosomal RNA 18s was used as an endogenous control. Error bars indicate standard error. *, $p \leq 0.1$; **, $p \leq 0.05$. n=3.

**Figure 15.** Histological sections of HSOC, treated with the described combinations of phytochemicals and irradiated with UVB, stained with hematoxylin-eosin (H-E) and visualized using bright-field microscopy.

**Claims**

1. A composition capable of sequential photonic transfer (SPT), wherein the composition comprises:

   • at least one organic compound capable of absorbing UVB/A light (280-400 nm) and emitting blue/green light (450-570 nm),
   wherein the at least one organic compound capable of absorbing UVB/A light (280-400 nm) and emitting blue/green light (450-570 nm) is selected from the group consisting of polyphenols, flavonoids, cannabinoids, and combinations thereof,
   • at least one organic compound capable of absorbing blue/green light (450-570 nm) and emitting red light (620-780 nm),
   wherein the at least one organic compound capable of absorbing blue/green light (450-570 nm) and emitting red light (620-780 nm) is selected from the group consisting of carotenoids, xanthenes, chlorophylls, oleic acids, and combinations thereof, and

• at least one photosensitive compound or a precursor thereof capable of absorbing red light and stimulating the production of ROS.

2. The composition according to claim 1, wherein the at least one organic compound capable of absorbing UVB/A light (280-400 nm) and emitting blue/green light (450-570 nm) is selected from the group consisting of morin, quercetin, cannabidiol, cannabinol, cannabichromene, and combinations thereof.

3. The composition according to any one of claims 1 or 2, wherein the at least one organic compound capable of absorbing blue/green light (450-570 nm) and emitting red light (620-780 nm) is selected from the group consisting of chlorophyll A, chlorophyll B, chlorophyll C, chlorophyll D, chlorophyll F, and combinations thereof.

4. The composition according to any one of claims 1 to 3, wherein the at least one photosensitive compound or precursor thereof capable of absorbing red light and stimulating the production of ROS is Protoporphyrin IX (PpIX), aminolevulinic acid (ALA), and/or methyl-aminolevulinate (m-ALA).

5. The composition according to any one of claims 1 to 4, wherein the composition comprises:

• morin and/or cannabidiol,
• chlorophyll A, and
• methyl-aminolevulinate (m-ALA).

6. The composition according to any one of claims 1 to 5, wherein the composition is incorporated into a carrier system or a cosmetically or pharmaceutically acceptable sustained-release system selected from the group consisting of liposomes, mixed liposomes, oleosomes, niosomes, ethosomes, millicapsules, microcapsules, nanocapsules, sponges, cyclodextrins, vesicles, micelles, mixed surfactant micelles, phospholipid-surfactant mixed micelles, millispheres, microspheres, nanospheres, lipospheres, microemulsions, nanoemulsions, miniparticles, milliparticles, microparticles, nanoparticles, solid lipid nanoparticles, and nanostructured lipid carriers.

7. The composition according to any one of claims 1 to 6, wherein the composition is presented in a formulation selected from the group consisting of creams, multiple emulsions, anhydrous compositions, aqueous dispersions, oils, milks, balms, foams, lotions, gels, cream gels, hydroalcoholic solutions, hydroglycolic solutions, hydrogels, liniments, serums, soaps, shampoos, conditioners, serums, ointments, mousses, pomades, powders, bars, pencils, sprays, and aerosols.

8. The composition according to any one of claims 1 to 6, wherein the composition is incorporated into a product selected from the group consisting of sunscreens, under-eye correctors, foundations, makeup removers, cleansing milks, eyeshadows, lipsticks, lip glosses, lip protectors, and powders.

9. A composition according to any one of claims 1 to 8 for use as a medicament.

10. A composition according to any one of claims 1 to 8 for use in the treatment of skin defects, imperfections, or unaesthetic conditions, including fine or deep wrinkles, stretch marks, crow's feet, under-eye circles, hair loss, spider veins, hyperpigmentation, hypopigmentation, discoloration, age spots, loss of skin radiance, freckles, blemishes, tissue fragility, dryness, cracks, tactile roughness, acne, rosacea, atopic dermatitis, psoriasis, and eczema.

11. A composition according to any one of claims 1 to 8 for use in the treatment of the effects of solar radiation on human skin, including burns and sunspots.

12. Non-therapeutic use of a composition according to any one of claims 1 to 8 as a skin photoprotector.

13. A non-therapeutic method for skin photoprotection comprising topically applying a composition according to any one of claims 1 to 8.

**Patentansprüche**

1. Zusammensetzung, die zum sequentiellen photonischen Transfer (SPT) fähig ist, wobei die Zusammensetzung Folgendes umfasst:

- mindestens eine organische Verbindung, die in der Lage ist, UVB/A-Licht (280-400 nm) zu absorbieren und blau/grünes Licht (450-570 nm) zu emittieren,

wobei die mindestens eine organische Verbindung, die in der Lage ist, UVB/A-Licht (280-400 nm) zu absorbieren und blaues/grünes Licht (450-570 nm) zu emittieren, aus der Gruppe bestehend aus Polyphenolen, Flavonoiden, Cannabinoiden und Kombinationen davon ausgewählt ist,

- mindestens eine organische Verbindung, die in der Lage ist, blaues/grünes Licht (450-570 nm) zu absorbieren und rotes Licht (620-780 nm) zu emittieren,

wobei die mindestens eine organische Verbindung, die in der Lage ist, blaues/grünes Licht (450-570 nm) zu absorbieren und rotes Licht (620-780 nm) zu emittieren, aus der Gruppe bestehend aus Carotinoiden, Xanthenen, Chlorophyllen, Ölsäuren und Kombinationen davon ausgewählt ist, und

- mindestens eine lichtempfindliche Verbindung oder einen Vorläufer davon, der in der Lage ist, rotes Licht zu absorbieren und die Produktion von ROS zu stimulieren.

2. Zusammensetzung nach Anspruch 1, wobei die mindestens eine organische Verbindung, die in der Lage ist, UVB/A-Licht (280-400 nm) zu absorbieren und blaues/grünes Licht (450-570 nm) zu emittieren, aus der Gruppe bestehend aus Morin, Quercetin, Cannabidiol, Cannabinol, Cannabichromen und Kombinationen davon ausgewählt ist.

3. Zusammensetzung nach einem der Ansprüche 1 oder 2, wobei die mindestens eine organische Verbindung, die in der Lage ist, blaues/grünes Licht (450-570 nm) zu absorbieren und rotes Licht (620-780 nm) zu emittieren, aus der Gruppe bestehend aus Chlorophyll A, Chlorophyll B, Chlorophyll C, Chlorophyll D, Chlorophyll F und Kombinationen davon ausgewählt ist.

4. Zusammensetzung nach einem der Ansprüche 1 bis 3, wobei die mindestens eine lichtempfindliche Verbindung oder ein Vorläufer davon, die in der Lage sind, rotes Licht zu absorbieren und die Produktion von ROS zu stimulieren, Protoporphyrin IX (PpIX), Aminolävulinsäure (ALA) und/oder Methyl-Aminolävulinat (m-ALA) ist.

5. Zusammensetzung nach einem der Ansprüche 1 bis 4, wobei die Zusammensetzung Folgendes umfasst:

- Morin und/oder Cannabidiol,
- Chlorophyll A und
- Methyl-Aminolävulinat (m-ALA).

6. Zusammensetzung nach einem der Ansprüche 1 bis 5, wobei die Zusammensetzung in ein Trägersystem oder ein kosmetisch oder pharmazeutisch annehmbares System mit anhaltender Freisetzung eingearbeitet ist, das aus der Gruppe bestehend aus Liposomen, gemischten Liposomen, Oleosomen, Niosomen, Ethosomen, Millikapseln, Mikrokapseln, Nanokapseln, Schwämmen, Cyclodextrinen, Vesikeln, Mizellen, gemischten Tensidmizellen, gemischten Phospholipid-Tensid-Mizellen, Millikugeln, Mikrokugeln, Nanokugeln, Lipokugeln, Mikroemulsionen, Nanoemulsionen, Minipartikeln, Millipartikeln, Mikropartikeln, Nanopartikeln, festen Lipid-Nanopartikeln und nanostrukturierten Lipidträgern ausgewählt ist.

7. Zusammensetzung nach einem der Ansprüche 1 bis 6, wobei die Zusammensetzung in einer Formulierung dargeboten wird, die aus der Gruppe ausgewählt ist, bestehend aus Cremes, Mehrfachemulsionen, wasserfreien Zusammensetzungen, wässrigen Dispersionen, Ölen, Milchzubereitungen, Balsamen, Schäumen, Lotionen, Gelen, Cremegelen, hydroalkoholischen Lösungen, hydroglykolischen Lösungen, Hydrogelen, Einreibemitteln, Seren, Seifen, Shampoos, Conditioner, Seren, Salben, Mousses, Pomaden, Pulvern, Riegeln, Stifte, Sprays und Aerosolen.

8. Zusammensetzung nach einem der Ansprüche 1 bis 6, wobei die Zusammensetzung in ein Produkt eingearbeitet ist, das aus der Gruppe ausgewählt ist, bestehend aus Sonnenschutzmitteln, Unteraugen-Korrektoren, Grundierungen, Make-up-Entfernern, Reinigungsmilchen, Lidschatten, Lippenstiften, Lippen-Gloss, Lippenschutzmitteln und Pulvern.

9. Zusammensetzung nach einem der Ansprüche 1 bis 8 zur Verwendung als Arzneimittel.

10. Zusammensetzung nach einem der Ansprüche 1 bis 8 zur Verwendung bei der Behandlung von Defekten, Unvollkommenheiten oder unschönen Zuständen der Haut, einschließlich feiner oder tiefer Falten, Dehnungsstreifen, Krähenfüße, Augenringe, Haarausfall, Besenreiser, Hyperpigmentierung, Hypopigmentierung, Verfärbungen, Altersflecken, Verlust der Hautausstrahlung, Sommersprossen, Unreinheiten, Gewebebrüchigkeit, Trockenheit, Risse, fühlbare Rauheit, Akne, Rosazea, atopische Dermatitis, Psoriasis und Ekzeme.

**11.** Zusammensetzung nach einem der Ansprüche 1 bis 8 zur Verwendung bei der Behandlung der Auswirkungen von Sonnenstrahlung auf die menschliche Haut, einschließlich Verbrennungen und Sonnenflecken.

**12.** Nichttherapeutische Verwendung einer Zusammensetzung nach einem der Ansprüche 1 bis 8 als Lichtschutzmittel für die Haut.

**13.** Nichttherapeutisches Verfahren zum Lichtschutz der Haut, umfassend das topische Auftragen einer Zusammensetzung nach einem der Ansprüche 1 bis 8.

## Revendications

**1.** Composition capable de réaliser le transfert photonique séquentiel (TFS), dans laquelle la composition comprend:

• au moins un composé organique capable d'absorber de la lumière UVB/A (280-400 nm) et d'émettre de la lumière bleue/verte (450-570 nm),
dans laquelle l'au moins un composé organique capable d'absorber de la lumière UVB/A (280-400 nm) et d'émettre de la lumière bleue/verte (450-570 nm) est choisi dans le groupe constitué par les polyphénols, les flavonoïdes, les cannabinoïdes et les combinaisons de ceux-ci,
• au moins un composé organique capable d'absorber de la lumière bleue/verte (450-570 nm) et d'émettre de la lumière rouge (620-780 nm),
dans laquelle l'au moins un composé organique capable d'absorber de la lumière bleue/verte (450-570 nm) et d'émettre de la lumière rouge (620-780 nm) est choisi dans le groupe constitué par les carotenoïdes, les xanthènes, les chlorophylles, les acides oléiques et les combinaisons de ceux-ci, et
• au moins un composé photosensible ou un précurseur de celui-ci capable d'absorber de la lumière rouge et de stimuler la production de ROS.

**2.** Composition selon la revendication 1, dans laquelle l'au moins un composé organique capable d'absorber de la lumière UVB/A (280-400 nm) et d'émettre de la lumière bleue/verte (450-570 nm) est choisi dans le groupe constitué par la morine, la quercétine, le cannabidiol, le cannabinol, le cannabichromène et les combinaisons de ceux-ci.

**3.** Composition selon l'une quelconque des revendications 1 ou 2, dans laquelle l'au moins un composé organique capable d'absorber de la lumière bleue/verte (450-570 nm) et d'émettre de la lumière rouge (620-780 nm) est choisi dans le groupe constitué par la chlorophylle A, la chlorophylle B, la chlorophylle C, la chlorophylle D, la chlorophylle F et les combinaisons de celles-ci.

**4.** Composition selon l'une quelconque des revendications 1 à 3, dans laquelle l'au moins un composé photosensible ou précurseur de celui-ci capable d'absorber de la lumière rouge et de stimuler la production de ROS est la protoporphyrine IX (PpIX), l'acide aminolevulinique (ALA) et/ou l'aminolevulinate de méthyle (m-ALA).

**5.** Composition selon l'une quelconque des revendications 1 à 4, dans laquelle la composition comprend:

• de la morine et/ou du cannabidiol,
• de la chlorophylle A, et
• de l'aminolevulinate de méthyle (m-ALA).

**6.** Composition selon l'une quelconque des revendications 1 à 5, dans laquelle la composition est incorporée dans un système vecteur ou un système à libération prolongée cosmétiquement ou pharmaceutiquement acceptable choisi dans le groupe constitué par les liposomes, les liposomes mixtes, les oléosomes, les niosomes, les éthosomes, les millicapsules, les microcapsules, les nanocapsules, les éponges, les cyclodextrines, les vésicules, les micelles, les micelles de tensioactifs mixtes, les micelles mixtes de phospholipides-tensioactifs, les millisphères, les microsphères, les nanosphères, les liposphères, les microémulsions, les nanoémulsions, les miniparticules, les milliparticules, les microparticules, les nanoparticules, les nanoparticules de lipides solides et les transporteurs lipidiques nanostructurés.

**7.** Composition selon l'une quelconque des revendications 1 à 6, dans laquelle la composition est présentée dans une formulation choisie dans le groupe constitué par les crèmes, les émulsions multiples, les compositions anhydres, les dispersions aqueuses, les huiles, les laits, les baumes, les écumes, les lotions, les gels, les gels-crèmes, les solutions hydroalcooliques, les solutions hydroglycoliques, les hydrogels, les liniments, les sérums, les savons, les shampoings, les après-shampoings, les sérums, les onguents, les mousses, les pommades, les poudres, les barres, les crayons, les vaporisateurs et les aérosols.

8. Composition selon l'une quelconque des revendications 1 à 6, dans laquelle la composition est incorporé dans un produit choisi dans le groupe constitué par les crèmes solaires, les correcteurs sous les yeux, les fonds de teint, les démaquillants, les laits de nettoyage, les fards à paupières, les rouges à lèvres, les brillant à lèvres, les protecteurs des lèvres et les poudres.

9. Composition selon l'une quelconque des revendications 1 à 8 pour son utilisation en tant que médicament.

10. Composition selon l'une quelconque des revendications 1 à 8 pour son utilisation dans le traitement des défauts, des imperfections ou des affections inesthétiques cutanés, y compris les rides fines ou profondes, les vergetures, les pattes d'oie, les cernes, la perte de cheveux, les varicosités, l'hyperpigmentation, l'hypopigmentation, la décoloration, les taches de vieillesse, la perte d'éclat cutané, les taches de rousseur, les imperfections, la fragilité tissulaire, la sécheresse, les fissures, la rugosité tactile, l'acné, la rosacée, la dermatite atopique, le psoriasis et l'eczéma.

11. Composition selon l'une quelconque des revendications 1 à 8 pour son utilisation dans le traitement des effets du rayonnement solaire sur la peau humaine, y compris les brûlures et les taches solaires.

12. Utilisation non thérapeutique d'une composition selon l'une quelconque des revendications 1 à 8 en tant que photoprotecteur de la peau.

13. Procédé non thérapeutique pour la photoprotection de la peau comprenant l'application topique d'une composition selon l'une quelconque des revendications 1 à 8.

**Fig. 1**

**Fig. 2**

Fig. 3

**Fig. 4**

**Fig. 5**

## Fig. 6

## Fig. 7

## Fig. 8

Fig. 9
**Cell death** quantification
by flow cytometry

Fig. 10

Fig. 11
**Cytotoxicity** associated with LDH

## Fig. 12

**Cell proliferation** quantification by flow cytometry

## Fig. 13

## Fig. 14

Gene expression by qRT-PCR of **extracellular matrix** markers

**Fig. 15**

## REFERENCES CITED IN THE DESCRIPTION

### Patent documents cited in the description

- WO 2009095568 A2

- WO 2008106983 A1

### Non-patent literature cited in the description

- **NG W** ; **IKEDA S**. Standardized, Defined Serum-Free Culture of a Human Skin Equivalent on Fibroblast-Populated Collagen Scaffold.. *Acta Derm Venereol.*, 2011, vol. 91, 387-91
- **FEOKTISTOVA, M.** ; **GESERICK, P.** ; **LEVERKUS, M.** Crystal Violet Assay for Determining Viability of Cultured Cells. *Cold Spring Harbor Protocols*, 2016, vol. 2016 (4)
- **RICCARDI, C.** ; **NICOLETTI, I.** Analysis of apoptosis by propidium iodide staining and flow cytometry.. *Nature Protocols*, 2006, vol. 1 (3), 1458-1461
- **YUAN, J.** ; **ADAMSKI, R.** ; **CHEN, J.** Focus on histone variant H2AX: to be or not to be. *FEBS letters*, 2010, vol. 584 (17), 3717-3724
- **ANA CELESTE XIMENES OLIVEIRA** ; **ISMAEL ÁNGEL RODRIGUEZ** ; **INGRID GARZÓN** ; **MIGUEL ALAMINOS**. Estudio de viabilidad celular en un modelo experimental de fibroblastos gingivales humanos cultivados en ausencia de suero bovino fetal. *Actual. Med.*, May 2010, vol. 95 (780), 013-018
- **CARRASCO, E** ; **BLÁZQUEZ-CASTRO, A.** ; **CALVO, M. I.** ; **JUARRANZ, Á.** ; **ESPADA, J.** Switching on a transient endogenous ROS production in mammalian cells and tissues. *Methods (San Diego, Calif.)*, 2016, vol. 109, 180-189

- **IMRAY, P.** ; **MANGAN, T.** ; **SAUL, A.** ; **KIDSON, C.** Effects of ultraviolet irradiation on the cell cycle in normal and UV-sensitive cell lines, with reference to the nature of the defect in xeroderma pigmentosum variant.. *Mutation research*, 1983, vol. 112 (5), 301-309
- **RITTIÉ, L.** ; **FISHER, G. J.** Natural and sun-induced aging of human skin. *Cold Spring Harbor perspectives in medicine*, 2015, vol. 5 (1), a015370
- **MENDOZA-GARCIA, J.** ; **SEBASTIAN, A.** ; **ALONSO-RASGADO, T.** ; **BAYAT, A.** Optimization of an ex vivo wound healing model in the adult human skin: Functional evaluation using photodynamic therapy. *Wound repair and regeneration: official publication of the Wound Healing Society [and] the European Tissue Repair Society*, 2015, vol. 23 (5), 685-702
- **MASUMA, R.** ; **KASHIMA, S.** ; **KURASAKI, M.** ; **OKUNO, T.** Effects of UV wavelength on cell damages caused by UV irradiation in PC12 cells.. *Journal of photochemistry and photobiology. B, Biology*, 2013, vol. 125, 202-208